# EUROPÄISCHE PATENTSCHRIFT

(11) **EP 1 208 183 B1**
(45) Veröffentlichungstag und Bekanntmachung des Hinweises auf die Patenterteilung: **26.10.2005**
(21) Anmeldenummer: 00956115.0
(22) Anmeldetag: 01.08.2000
(51) Int. Cl.: C10G 70/04

(54) **VERFAHREN UND ANLAGE ZUM THERMISCHEN SPALTEN VON KOHLENWASSERSTOFFEN, INSBESONDERE ZUR HERSTELLUNG VON OLEFINEN**
METHOD AND INSTALLATION FOR THERMALLY CRACKING HYDROCARBONS, ESPECIALLY FOR PRODUCING OLEFINS
PROCEDE ET INSTALLATION DE CRAQUAGE THERMIQUE D'HYDROCARBURES, EN PARTICULIER POUR LA PRODUCTION D'OLEFINES

(30) Priorität: 03.08.1999 DE 19936548
(43) Veröffentlichungstag der Anmeldung: 29.05.2002
(73) Patentinhaber: ALSTOM Power Energy Recovery GmbH, 34123 Kassel-Bettenhausen (DE)
(72) Erfinder: JEKERLE, Jiri, D-34225 Baunatal (DE); KÖSTERS, Peter, Hubertus, NL-4571 SR Axel (NL)
(86) Internationale Anmeldenummer: PCT/DE2000/002562
(87) Internationale Veröffentlichungsnummer: WO 2001/009269

(56) Entgegenhaltungen:
- EP-A- 0 031 609
- WO-A-99/31201
- DE-C- 19 824 575
- US-A- 4 143 521

## Beschreibung

Die Erfindung betrifft ein Verfahren zum thermischen Spalten von Kohlenwasserstoffen, insbesondere zur Herstellung von Olefinen gemäß dem Oberbegriff des Anspruches 1 sowie eine Anlage zum thermischen Spalten von Kohlenwasserstoffen, insbesondere zur Herstellung von Olefinen gemäß dem Oberbegriff des Anspruches 6.

Ethylen, Propylen, Butadien und andere Olefine bilden die Basis der modernen organischen Chemieindustrie und sind damit die Grundlage für eine ganze Reihe von chemischen Produkten, wie Kunststoffe, Pharmazie- und Kosmetikprodukten etc.

Die Herstellung der Olefine erfolgt u. a. in bekannter Weise in thermischen Spaltanlagen (Steamcracker) durch thermische Spaltung von gasförmigen Einsatzstoffen, wie Ethan, Propan, LPG, oder flüssigen Einsatzstoffen, wie Naphtha, Gasöl und vorbehandelten Produkten aus Hydrocrockern. Der Spaltprozeß läuft in den Rohrschlangen eines durch Boden- oder Wandbrenner beheizten Röhrenspaltofens ab. Ein Gemisch von Kohlenwasserstoffen und Wasserdampf wird schnell auf eine Temperatur von 750 °C bis 900 °C aufgeheizt. Die bei der Spoltreaktion gebildeten Spaltgase sind bei der hohen Spalttemperatur chemisch instabil und müssen in einem sogenannten Spaltgaskühler sehr schnell innerhalb von Millisekunden unter die kritische Temperatur von etwa 650 °C abgekühlt werden. Das abgekühlte Spaltgas enthält neben den gewünschten Olefinen auch andere Kohlenwasserstoffe und einen beträchtlichen Anteil an Wasserstoff. Nach der rapiden Abkühlung im Spaltgaskühler erfolgt bei Verwendung von flüssigen Einsatzstoffen (diese bedingen das Vorhandensein von schweren Produkten im Spaltgas) ein Abkühlen, z. B. durch Einspritzkühler, des Spaltgases in einer Quencheinrichtung und ein nachfolgendes Einleiten in Trennkolonnen für Benzin und Schweröl, wobei die schweren Produkte durch die vorgenannten Komponenten aus dem Spaltgas abgetrennt werden. Anschließend bzw. bei Verwendung von gasförmigen Einsatzstoffen wird das Spaltgas in eine Quenchkolonne zwecks Entfernung des Wasserdampfes geleitet, in weiterer Folge durch einen Kompressor in der Regel auf etwa 36 bar verdichtet, in einem Trockner von Restwasser befreit, in einer Tiefkühlanlage abgekühlt und in mehrere verschiedene Trennkolonnen geführt, wobei in der Tiefkühlanlage der Wasserstoff und in den Trennkolonnen einzelne Produkte, wie beispielsweise Ethylen und Propylen, abgetrennt werden. Der Volumenanteil des Wasserstoffes beträgt nach der Abkühlung des Spaltgases im Spaltgaskühler je nach Einsatzprodukt bzw. -stoff 4 % bis 25 % des Spaltgasvolumens.

Bei diesem bekannten Verfahren bzw. dieser bekannten Anlage zum thermischen Spalten von Kohlenwasserstoffen, insbesondere zur Herstellung von Olefinen, hat sich nachteilig gezeigt, daß der hohe Anteil an Wasserstoff im Spaltgas einen wesentlichen Teil der Kompressor leistung verbraucht und infolge des hohen Volumendurchsatzes die Quenchkolonne für Wasser und, falls vorhanden, die Trennkolonnen für Benzin und Schweröl belasten.

Durch Druckschrift WO 99/31201 A ist ein Verfahren zur Gewinnung von Olefinen, insbesondere ein verbessertes Verfahren zum Abtrennen von Olefinen aus Olefine und Wasserstoff enthaltenden Gasen bekannt geworden, das eine Kombination aus einer Membranabscheidung und einer Druckwechseladsorptionstechnik (auch PSA: Pressure Swing Adsorption) benutzt. Das im Spaltgasofen (Cracking oven) gewonnene Spaltgas bzw. Cracking effluent wird nach einer Reihe von Verfahrensschritten (Quencher, Primary Separation Section, Scrubber und Dryer) und mindestens einer Kompressorstufe einem Membranabscheider zugeführt, aus dem ein wasserstoffreicher Permeatgasstrom und ein wasserstoffarmer Retentatgasstrom gewonnen wird. Der wasserstoffreiche Permeatgasstrom wird anschließend über ggf. notwendige zusätzliche Kompressoren einem Druckwechseladsorptions-System zugeführt, mittels dem ein wasserstoffreicher, nichtadsorbierter Strom und ein Kohlenwasserstoffprodukte enthaltender, adsorbierter Strom erzeugt wird. Während der wasserstoffreiche Strom einer Hydrierung zugeführt wird, wird der Kohlenwasserstoffprodukte enthaltende Strom über einen Kompressor wieder dem Membranabscheider zugeführt, um weiteren Wasserstoff aus dem adsorbierten Strom abzuscheiden und um einen kohlenwasserstoffreicheren Gasstrom als Retentat zu erzeugen. Das Retentat wird anschließend einer Cryogenic Separation Section zugeführt, in der Methan von den schwereren Kohlenwasserstoffprodukten abgeschieden wird und letztere weiteren Fractionation Columns zugeführt wird.

Durch Druckschrift US 5,082,481 ist ein Membrane Separation Process for Cracked Gases bekannt geworden. Bei dem bekannt gewordenen Verfahren wird Wasserstoff, Kohlendioxid und Wasser aus einem wenigstens Olefine enthaltenden Spaltgas gewonnen. Das Verfahren umfasst das Komprimieren des Spaltgases in wenigstens einer Kompressorstufe, das Abtrennen wenigstens eines Teils von Wasserstoff, Kohlendioxid und Wasser aus dem komprimierten Spaltgas vor dem Abkühlen des Spaltgases und darauffolgend das Abkühlen des Spaltgases um eine Niedrigtemperatur-Abtrennung der niedrigsiedenden Bestandteile des Spaltgases zu bewirken. Das Abtrennen wenigstens eines Teils von Wasserstoff, Kohlendioxid und Wasser aus dem komprimierten Spaltgas kann durch eine semipermeable Membran bewirkt werden.

Es ist Aufgabe der vorliegenden Erfindung, ein Verfahren und eine Anlage zum thermischen Spalten von Kohlenwasserstoffen, insbesondere zur Herstellung von Olefinen, zu schaffen, das bzw. die die vorgenannten Nachteile vermeidet.

Die Aufgabe wird bezüglich des Verfahrens gemäß den kennzeichnenden Merkmalen des Patentanspruches 1 und bezüglich der Anlage gemäß den kennzeichnenden Merkmalen des Patentanspruches 6 gelöst.

Dabei wird bei dem erfindungsgemäßen Verfahren in Strömungsrichtung des Prozeßmediums gesehen stromaufwärts des Kompressors Wasserstoffes aus dem Spaltgas extrahiert bzw. bei der erfindungsgemäßen Anlage in Strömungsrichtung des Prozeßmediums gesehen stromaufwärts des Kompressors ein nach dem Prinzip der Membrandiffusion arbeitender Stoffaustauscher zur selektiven Extraktion von Wasserstoff aus dem Spaltgas angeordnet.

Ein Vorteil ist, dass die Baugröße des Kompressors kleiner und daher Kostengünstiger austallen kann und weniger Energieverbrauch und damit Kosten verursacht oder bei gleichbleibender Baugröße ein vergrößertes Spaltgas-Volumen durchsatz 'H' ermöglicht wird. Vorteilhaft ist ferner, dass auch alle weiteren Komponenten stromabwärts des Kompressors wegen des kleineren Durchsatzvolumens kleiner u. Kostengünstiger ausfallen können bzw. ein vergrößerter Spaltgas-Volumendurchsatz möglich ist.

Durch den Einsatz des einen Teil des Wasserstoffes extrahierenden Stoffaustauschers und bei Ausnutzung der vollen Kompressorleistung kann die Spaltgasmenge und somit auch die Olefinproduktion gesteigert werden. Das gleiche gilt für alle anderen Apparate am kalten Ende der thermischen Spaltanlage. Ein zusätzlicher Vorteil des erfindungsgemäßen

Verfahrens bzw. der erfindungsgemäßen Anlage kann die Gewinnung von sehr reinem Wasserstoff sein, der sehr gute Marktchancen hat.

In bevorzugter Weise wird der Wasserstoff aus dem Spaltgas in Strömungsrichtung des Prozeßmediums gesehen innerhalb der thermischen Spaltanlage unmittelbar nach der Abkühlung des Spaltgases. im Spaltgaskühler extrahiert. Durch diese Maßnahme wird das Gasvolumen stromabwärts des Spaltgaskühlers für alle Komponenten kleiner, d. h. die Komponenten können kleiner dimensioniert werden bzw. bei ursprünglicher Größe ist ein erhöhter Spaltgasdurchsatz möglich.

In vorteilhafter Ausgestaltung des erfindungsgemäßen Verfahrens reduziert ein Spülmedium den Partialdruck des extrahierten Wasserstoffes und führt diesen ab. Besonders vorteilhaft kann es sein, daß als Spülmedium ein Heiz- oder Kühlmedium eingesetzt wird, das gleichzeitig mit der Extraktion des Wasserstoffes aus dem Spaltgas durch indirekten Wärmetausch eine Erwärmung oder Abkühlung des Spaltgases bewirkt. Durch die Reduzierung des Portialdruckes des Wasserstoffes wird eine verbesserte Wasserstoffdiffusion durch die Membranwand erreicht und die Effizienz der Wasserstoffabtrennung und somit auch der Wirkungsgrad der Gesamtanlage erhöht.

Bei der erfindungsgemäßen Anlage ist der Stoffaustauscher bevorzugt unmittelbar dem Spaltgaskühler nachgeschaltet.

In zweckmäßiger Ausbildung weist der Stoffaustauscher der erfindungsgemäßen Anlage mindestens einen Strömungskanal und jeweils mindestens einen Ein- und Austritt für das Spaltgas, mindestens einen Strömungskanal und jeweils mindestens einen Ein- und Austritt für ein Spülmedium, eine das Spaltgas und das Spülmedium trennende und den Strömungskanälen für das Spaltgas und das Spülmedium gemeinsame Membranwand, die mindestens einen Teil der Wandung des Strömungskanals für das Spaltgas und das Spülmedium bildet, auf, wobei zumindest eine Sektion der Membranwand wasserstoffdiffundierbar ausgebildet ist. Dabei ist die Membrane bzw. die Membranwand (bzw. Membranwände bei mehreren Strömungskanälen für Spaltgas und/oder Spülmedium) des Stoffoustauschers in bevorzugter Weise aus keramischem Material ausgebildet. In weiterer bevorzugter Ausbildung ist die aus keramischem Material gebildete Membrane bzw. Membranwand aus einem makroporösen Material auf der Basis von Aluminiumoxid oder einer anderen Oxidkeramik (Trägerschicht) und einer mikroporösen Schicht auf Siliciumbasis oder einer anderen Basis gebildet. Dabei kann die mikroporöse Schicht als Katalysator ausgebildet sein, um über einen katalytischen Prozeß auf das Spaltgas einwirken zu können. Beispielsweise kann eine Hydrierung der doppelten Olefine (Azetylene) mit Hilfe von Palladium bewirkt werden.

In vorteilhafter Ausbildung der erfindungsgemäßen Anlage ist der Stoffaustauscher mit einem Heiz- oder Kühlmedium als Spülmedium betreibbar. Dabei findet gleichzeitig ein indirekter Wärmetausch zwischen dem Spaltgas und dem Heiz- oder Kühlmedium und ein Stoffaustausch in Form der Extraktion des Wasserstoffes aus dem Spaltgas statt. Der extrahierte Wasserstoff wird durch das Heiz- oder Kühlmedium entfernt, wobei das Heiz- oder Kühlmedium ebenso wie das Spülmedium den Partialdruck des Wasserstoffes herabsetzen kann.

Es ist ferner zweckmäßig, den Stoffaustauscher gasseitig mit einer Bypasseinrichtung vorzusehen, um an der erfindungsgemäßen Anlage zum thermischen Spalten von Kohlenwasserstoffen, insbesondere zur Herstellung von Olefinen, bei bestimmten Betriebsfällen, z. B. beim Entkoken, den Stoffaustauscher ausschalten und vom Gasstrom trennen zu können.

Die Erfindung soll anhand der Beschreibung und der Zeichnung näher erläutert werden.

Es zeigen
- Fig. 1: eine erfindungsgemäße thermische Spaltanlage, schematisch und vereinfacht dargestellt
- Fig. 2: einen Stoffaustauscher in Form eines Röhrenstoffaustauschers.

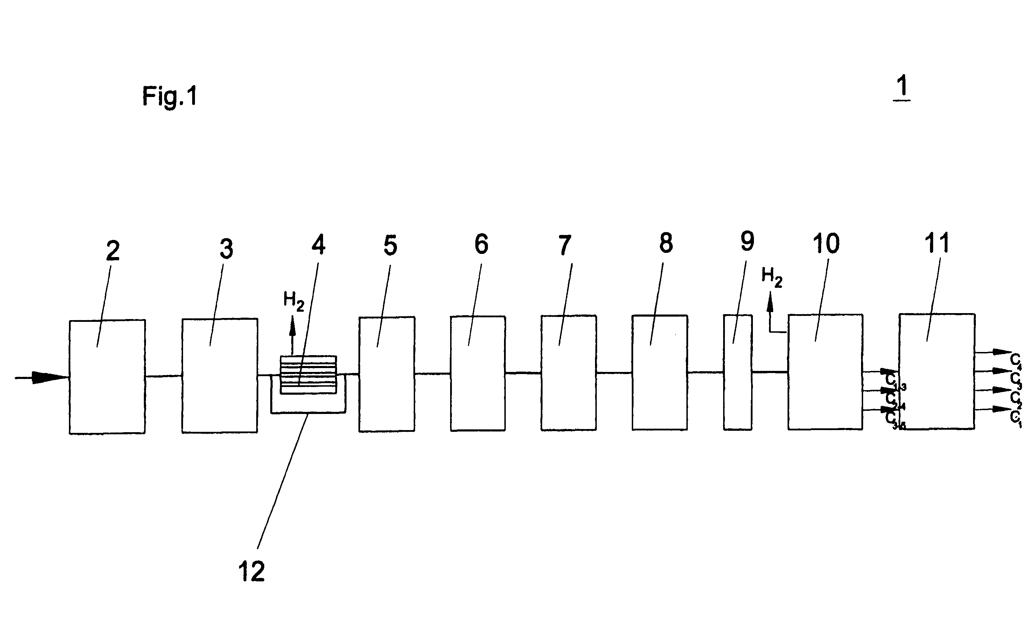

Fig. 1 zeigt schematisch und vereinfacht dargestellt die schrittweise Herstellung von Olefinen in einer thermischen Spoltanlage. Gasförmige Einsatzprodukte bzw. -stoffe, wie Ethan, Propan, LPG, oder flüssige Einsatzstoffe, wie Naphtha, Gasöl, sowie vorbehandelte Produkte aus Hydrocrackern werden einem Röhrenspaltofen 2 der thermischen Spaltanlage 1 zugeführt und darin thermisch gespalten. Im Röhrenspaltofen 2 wird der Kohlenwasserstoff (der Einsatzstoff) gemeinsam mit zugemischtem Wasserdampf (aus einer externen oder internen Dompfproduktion kommend) schnell auf eine Temperatur von 750 °C bis 900 °C aufgeheizt, wobei der Spaltprozeß in den Rohrschlangen des durch Boden- oder Wandbrenner beheizten Röhrenspaltofens 2 abläuft. Das bei der Spoltreaktion im Röhrenspaltofen 2 gebildete Spaltgas ist bei der hohen Spalttemperatur chemisch instabil und wird in einem Spaltgaskühler 3 sehr schnell innerhalb von Millisekunden unter die kritische Temperatur von etwa 650 °C abgekühlt. Das abgekühlte Spaltgas enthält neben den gewünschten Olefinen auch andere Kohlenwasserstoffe und einen beträchtlichen Anteil an Wasserstoff. Je noch Einsatzstoff beträgt der Volumenanteil des Wasserstoffes am Spaltgas nach dem Spaltgaskühler 4 % bis 25 %.

Das gewonnene Spaltgas mit seinem Wasserstoffanteil wird unmittelbar nach dessen Austritt aus dem Spaltgaskühler 3 einem Stoffaustauscher 4 zugeführt. Der Stoffaustauscher 4, der nachfolgend beschrieben ist, entzieht bzw. extrahiert dem Spaltgas einen Teil des Wasserstoffes, wobei dem Spaltgas zweckmäßigerweise mindestens 30 % des gesamten Wasserstoffgehaltes extrahiert wird. Je noch Anordnungsstelle des Stoffaustauschers 4 bzw. nach Einsatzstoff bzw. noch Eigenschaft der Gasdiffusions-Membrane können Wasserstoffgehalte bis 50 % und weit darüber, gemessen am Gesamtwasserstoffgehalt, aus dem Spaltgas extrahiert werden.

Nach der rapiden Abkühlung im Spaltgaskühler 3 und der Extraktion eines Teiles des Wasserstoffes im Stoffaustauscher 4 erfolgt bei Verwendung von flüssigen Einsatzstoffen (diese bedingen das Vorhandensein von schweren Produkten im Spaltgas) ein Abkühlen, z. B. durch Einspritzkühler, des Spaltgases in einer Quencheinrichtung 5 und ein nachfolgendes Einleiten in Trennkolonnen 6 für Benzin und Schweröl, wobei die schweren Produkte durch die vorgenannten Komponenten aus dem Spaltgas abgetrennt werden. Anschließend bzw. bei Verwendung von gasförmigen Einsatzstoffen wird das Spaltgas in eine Quenchkolonne 7 zwecks Entfernung des Wasserdampfes geleitet, in weiterer Folge durch einen Kompressor 8 in der Regel auf etwa 36 bar verdichtet - zweckmäßigerweise auf mindestens 20 bar -, in einem Trockner 9 von Restwasser befreit, in einer Tiefkühlanlage 10 abgekühlt und in mehrere verschiedene Trennkolonnen 11 geführt, wobei in der Tiefkühlanlage 10 der restliche Wasserstoff und in den Trennkolonnen 11 einzelne Produkte, wie beispielsweise Ethylen und Propylen, abgetrennt werden.

Durch die Extraktion des Wasserstoffes aus dem Spaltgas verringert sich das Volumen des Spaltgases für die Anlagenteile stromabwärts des Stoffaustauschers 4 und sämtliche Komponenten stromabwärts des Stoffaustouschers 4 können entweder kleiner dimensioniert werden oder weisen nunmehr einen größeren Spaltgasdurchsatz auf. Die erfordertiche Verdichtungsleistung des Kompressors 8 wird in vorteilhafter Weise erheblich vermindert. Ferner wird durch die Verdichtung im Kompressor der Partialdruck des Wasserstoffes erhöht. Durch den Einsatz des Stoffaustauschers 4 und bei Ausnutzung der vollen Kompressorleistung kann die Spaltgasmenge und somit auch die Olefinproduktion gesteigert werden. Ein zusätzlicher Vorteil dieses Verfahrens bzw. dieser Anlage kann die Gewinnung von sehr reinem Wasserstoff sein, der sehr gute Marktchancen hat.

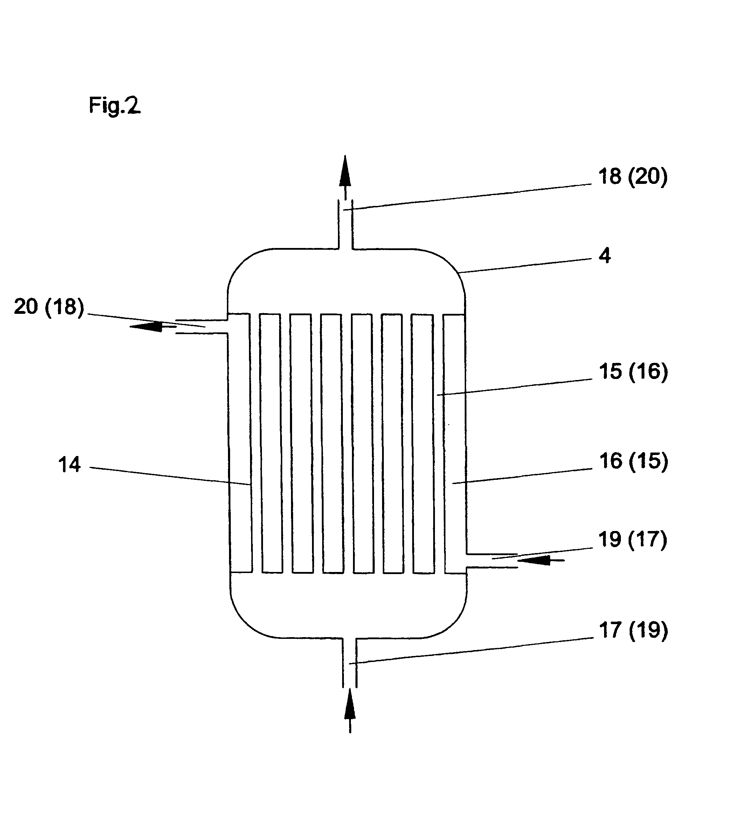

Zur Extraktion des Wasserstoffes kann der Stoffaustauscher 4, der ggf. auch als Massenaustauscher bezeichnet werden kann (siehe engl. "mass exchange"), aus einer Vielzahl von wasserstoffdiffundierbaren Membranen (Trennwände) bzw. Membranrohren 14 gebildet sein und beispielsweise gemäß Fig. 2 in Art und Weise wie ein Röhrenwärmeaustauscher ausgebildet und betrieben werden.

Derartige selektiv wasserstoffdiffundierbare Membranen bzw. Membranrohre 14 sind auf dem Markt bekannt. Sie ermöglichen die Abtrennung bzw. Extraktion des Wasserstoffes aus einem Gasgemisch nach dem Prinzip der Membrandiffusion (auch geläufig als Trennwanddiffusion bzw. Gasdiffusion bzw. Mikropordiffusion). Der Wasserstoff, der durch die Membranen bzw. Membranrohre 14 aufgrund des Partialdruckunterschiedes diffundiert, wird von einem Medium entfernt, das sich räumlich durch die Membranen bzw. Membranrohre 14 vom Spaltgas getrennt im Stoffaustauscher 4 befindet. Dieses Medium kann ein Spülmedium oder gleichzeitig ein das Spaltgas indirekt erwärmendes oder kühlendes Medium sein. Bei Verwendung von Stoffaustauschern 4 mit Membranrohren 14 kann das Spaltgas im oder außerhalb des Membranrohres 14 innerhalb des Stoffaustauschers 4 geführt werden.

Fig. 2 zeigt schematisch vereinfacht dargestellt beispielhaft einen Röhrenstoffaustauscher mit mehreren Membranrohren 14, die Strömungskanäle 15 bilden. Spaltgas strömt durch den Eintritt 17 und die Strömungskanäle 15 zum Austritt 18. Dabei diffundiert ein Teil des Wasserstoffes durch die Membranrohre 14 und wird durch ein Spülmedium in den Strömungskanälen 16 entfernt. Alternativ kann auch das Spülmedium durch die Membranrohre 14 und das Spaltgas durch die Strömungskanäle 16 geleitet werden (Positionen in Klammern).

Zweckmäßigerweise sind die Membranen bzw. Membranrohre 14 aus keramischem Werkstoff gebildet, da sich dieses Material für die vorliegende Anwendung als besonders geeignet erwiesen hat, da keramische Membranen gegenüber dem Prozeßmedium Spaltgas temperaturunempfindlich, basen- und säureunempfindlich und chemiebeständig sind. Die keramischen Membranen bzw. Membranrohre 14 können aus einem makroporösen Material auf der Basis von Aluminiumoxid oder einer anderen Oxidkeramik und einer mikroporösen Schicht, z. B. auf Siliciumbasis, erstellt werden. Die mikroporöse Schicht kann gleichzeitig als Katalysator wirken.

Erfindungsgemäß kann der Stoffaustauscher 4 beispielsweise zwischen der Quenchkolonne 7 und dem Kompressor 8 angeordnet werden. Durch die Abtrennung des Wasserdampfes in der Quenchkolonne 7 hat der Wasserstoff einen höheren Partialdruck und dies ermöglicht eine effizientere Diffusion des Wasserstoffes durch die Membranen bzw. Membranrohre 14 des Stoffaustauschers 4 bzw. den Einsatz eines kleineren Stoffaustauschers 4.

Für bestimmte Betriebsfälle, wie z. B. für das Entkoken der thermischen Spaltanlage 1, ist diese mit einer den Stoffaustauscher 4 gasseitig umgehenden Bypasseinrichtung 12 ausgebildet. Beispielsweise kann dann beim Entkoken der Stoffaustauscher 4 ausgeschaltet und der Gasstrom durch die Bypasseinrichtung 12 geleitet werden. Die Absperrung des Stoffaustauschers 4 kann beispielsweise mittels nicht näher dargestellter dichtschließender Schieber oder Klappen erfolgen.

Das den Wasserstoff entfernende Medium im Stoffaustauscher 4 kann nach der Mitnahme des Wasserstoffes anschließend gereinigt und im Kreislauf gefahren werden oder einer weiteren Bestimmung zugeführt werden.

### Bezugszeichenliste

- 1: Thermische Spaltanlage
- 2: Röhrenspaltofen
- 3: Spaltgaskühler
- 4: Stoffaustauscher
- 5: Quencheinrichtung (für Kondensieren der schweren Produkte)
- 6: Trennkolonnen (für Abtrennen von Schweröl und Benzin)
- 7: Quenchkolonne (für Entfernen von Wasserdampf)
- 8: Kompressor
- 9: Trockner
- 10: Tiefkühlanlage
- 11: Trennkolonnen (Destillationskolonnen)
- 12: Bypasseinrichtung
- 14: Membranrohr
- 15: Strömungskanal (für Spaltgas)
- 16: Strömungskanal (für Spül- bzw. Heiz- bzw. Kühlmedium)
- 17: Eintritt (für Spaltgas)
- 18: Austritt (für Spaltgas)
- 19: Eintritt (für Spül- bzw. Heiz- bzw. Kühlmedium)
- 20: Austritt (für Spül- bzw. Heiz- bzw. Kühlmedium)

## Patentansprüche

1. Verfahren zum thermischen Spalten von Kohlenwasserstoffen, insbesondere zur Herstellung von Olefinen, wobei in einem Spaltofen ein Gemisch von Kohlenwasserstoffen und Wasserdampf auf eine Temperatur von 750 °C bis 900 °C aufgeheizt und das dabei entstehende Wasserstoff enthaltende Spaltgas zeitlich unmittelbar noch der Aufheizung in einem Spaltgoskühler auf eine Temperatur unterhalb von ca. 650 °C abgekühlt, anschließend bei Vorhandensein von schweren Produkten durch Quenchen weiter abgekühlt und nachfolgend durch Abtrennung von Schweröl und Benzin befreit, anschließend aus dem leichte Produkte enthaltenden Spaltgas durch Quenchen Wasserdampf entfernt, durch einen Kompressor auf mindestens 20 bar verdichtet, durch Trocknung von Wasserresten befreit, in einer Tiefkühlanlage abgekühlt und zur Abtrennung der leichten Produkte in mehrere Trennkolonnen geführt wird, und wobei in Strömungsrichtung des Prozessmediums gesehen stromaufwärts der Tiefkühlanlage ein Teil des Wasserstoffes mittels selektiver Membrandiffusion aus dem Spaltgas extrahiert wird, **dadurch gekennzeichnet, dass** die Extraktion des Wasserstoffes aus dem Spaltgas in Strömungsrichtung des Prozessmediums gesehen stromaufwärts des Kompressors erfolgt.

2. Verfahren nach Anspruch 1, **dadurch gekennzeichnet, dass** die Extraktion des Wasserstoffes aus dem Spaltgas unmittelbar nach dessen Abkühlung im Spoltgoskühler erfolgt.

3. Verfahren nach einem der vorgenannten Ansprüche, **dadurch gekennzeichnet, dass** ein Spülmedium den Partialdruck des extrahierten Wasserstoffes reduziert und diesen entfernt.

4. Verfahren nach einem der vorgenannten Ansprüche, **dadurch gekennzeichnet, dass** als Spülmedium ein Heiz- oder Kühlmedium eingesetzt wird, das gleichzeitig mit der Extraktion des Wasserstoffes aus dem Spaltgas durch Wärmetausch eine Erwärmung oder Abkühlung des Spaltgases bewirkt.

5. Verfahren nach einem der vorgenannten Ansprüche, **dadurch gekennzeichnet, dass** das Spaltgas durch eine Bypasseinrichtung an der Einrichtung zur Extraktion des Wasserstoffes vorbeigeführt werden kann.

6. Anlage zum thermischen Spalten von Kohlenwasserstoffen, insbesondere zur Herstellung von Olefinen, welche in Strömungsrichtung des Prozessmediums gesehen
einen Spaltofen (2) zur Aufheizung eines Gemisches aus Kohlenwasserstoffen und Wasserdampf auf eine Temperatur von 750 °C bis 900 °C zur Erzeugung von Spaltgas,
einen Spaltgaskühler (3) zur Abkühlung des Spaltgases auf eine Temperatur unterhalb von ca. 650 °C,
bei Vorhandensein von schweren Produkten im Spaltgas eine Quencheinrichtung (5) zum Kondensieren der schweren Produkte und Trennkolonnen (6) zum Abscheiden von Schweröl und Benzin,
eine Quenchkolonne (7) zur Entfernung von Wasserdampf bzw. Wasser,
einen Kompressor (8) zur Verdichtung des Spaltgases auf mindestens 20 bar,
einen Trockner (9) zur Entfernung des Restwassers,
eine Tiefkühlanlage (10) und
mehrere Trennkolonnen (11) zur Abtrennung der leichten Produkte aufweist,
und wobei in Strömungsrichtung des Prozessmediums gesehen stromaufwärts der Tiefkühlanlage (10) ein nach dem Prinzip der Membrandiffusion arbeitender Stoffaustauscher (4) zur selektiven Extraktion von Wasserstoff aus dem Spaltgas angeordnet ist, **dadurch gekennzeichnet, dass** der Stoffaustauscher (4) in Strömungsrichtung des Prozessmediums gesehen stromaufwärts des Kompressors (8) angeordnet ist.

7. Anlage nach Anspruch 6, **dadurch gekennzeichnet, dass** der Stoffaustauscher (4) unmittelbar dem Spaltgaskühler (3) nachgeschaltet ist.

8. Anlage nach einem der Ansprüche 6 oder 7 **dadurch gekennzeichnet, dass** der Stoffaustauscher (4) mindestens einen Strömungskanal (15) und jeweils mindestens einen Ein- und Austritt (17, 18) für das Spaltgas, mindestens einen Strömungskanal (16) und jeweils mindestens einen Ein- und Austritt (19, 20) für ein Spülmedium, eine das Spaltgas und das Spülmedium trennende und den Strömungskanälen (15, 16) gemeinsame Membranwand (14), die mindestens einen Teil der Wandung des Strömungskanales (15, 16) bildet, aufweist, wobei zumindest eine Sektion der Membranwand (14) wasserstoffdiffundierbar ausgebildet ist.

9. Anlage nach Anspruch 8, **dadurch gekennzeichnet, dass** die Membrane bzw. Membranwand des Stoffaustauschers (4) aus keramischem Material gebildet ist.

10. Anlage nach Anspruch 9, **dadurch gekennzeichnet, dass** die aus keramischem Material gebildete Membrane bzw. Membranwand aus einem makroporösen Material auf der Basis von Aluminiumoxid oder einer anderen Oxidkeramik und einer mikroporösen Schicht auf Siliciumbasis oder einer anderen Basis gebildet sind.

11. Anlage nach Anspruch 10, **dadurch gekennzeichnet, dass** die mikroporöse Schicht des keramischen Materiales der Membrane bzw. der Membranwand gleichzeitig als Katalysator ausgebildet ist.

12. Anlage noch einem der Ansprüche 6 bis 11, **dadurch gekennzeichnet, dass** der Stoffaustauscher (4) mit einem Heiz- oder Kühlmedium als Spülmedium betreibbar ist.

13. Anlage nach einem der Ansprüche 6 bis 12, **dadurch gekennzeichnet, dass** der Stoffaustauscher (4) mit einer gasseitigen Bypasseinrichtung (12) ausgebildet ist.

## Claims

1. Process for steam cracking of hydrocarbons, especially for the production of olefins, which involves heating a mixture of hydrocarbons and steam in a cracking furnace to a temperature of 750° C to 900° C, and then, immediately following the heating in time, cooling the resulting cracking gas, which contains hydrogen, to a temperature below about 650° C in a cracking gas cooler, then, if heavy products are present, further cooling it by quenching, and then freeing it by separation of heavy oil and of gasoline, then quenching the cracking gas, which contains light products, to remove steam from it, compressing it to at least 20 bar with a compressor, freeing it of residual water by drying, cooling it in a deep cooling system, feeding it into several separation columns to separate the light products, and that upstream of the deep-cooling system, when viewed in the direction of flow of the process medium, part of the hydrogen is extracted from the cracking gas by means of selective membrane diffusion, **characterized by** the fact that the hydrogen is extracted from the cracking gas upstream of the compressor, when viewed in the direction of flow of the process medium.

2. Process according to Claim 1, **characterized by** the fact that the hydrogen is extracted from the cracking gas immediately after it is cooled.

3. Process according to one of the preceding claims, **characterized by** the fact that a flushing medium reduces the partial pressure of the extracted hydrogen and removes it.

4. Process according to one of the preceding claims, **characterized by** the fact that the flushing medium that is used is a heating or cooling medium which heats or cools the cracking gas through exchange of heat at the same time as it extracts hydrogen from the cracking gas.

5. Process according to one of the preceding claims, **characterized by** the fact that the cracking gas can bypass the device for extracting hydrogen through a bypass device.

6. System for steam cracking of hydrocarbons, especially for producing olefins, which has, when viewed in the direction of flow of the process medium:
a cracking furnace (2) for heating a mixture of hydrocarbons and steam to a temperature of 750° C to 900° C to produce cracking gas;
a cracking gas cooler (3) to cool off the cracking gas to a temperature below about 650° C;
if heavy products are present in the cracking gas, a quenching device (5) to condense the heavy products and separation columns (6) to separate heavy oil and gasoline;
a quenching column (7) to remove steam and water;
a compressor (8) to compress the cracking gas to at least 20 bar;
a drier (9) to remove the residual water;
a deep-cooling system (10);
several separation columns (11) to separate the light products; and
that it has arranged in it, upstream of deep-cooling system (10), when viewed in the direction of flow of the process medium, a mass transfer device (4) working according to the principle of membrane diffusion for selective extraction of hydrogen from the cracking gas, **characterized by** the fact that the mass transfer device (4) is arranged upstream of the compressor (8), when viewed in the direction of flow of the process medium.

7. System according to Claim 6, **characterized by** the fact that the mass transfer device (4) is immediately downstream of the cracking gas cooler (3).

8. System according to one of Claims 6 or 7, **characterized by** the fact that the mass transfer device (4) has at least one flow channel (15) and at least one inlet and outlet (17, 18) for the cracking gas, at least one flow channel (16) and at least one inlet and outlet (19, 20) for a flushing medium, a membrane wall (14) which separates the cracking gas from the flushing medium and is common to the flow channels (15, 16) and which forms at least one part of the wall of the flow channel (15, 16), with at least one section of the membrane wall (14) being made hydrogen-diffusible.

9. System according to Claim 8, **characterized by** the fact that the membranes or membrane wall of the mass transfer device (4) [are] made of ceramic material.

10. System according to Claim 9, **characterized by** the fact that the membrane or membrane wall made of ceramic material is made of a macroporous material on an aluminum oxide base or another oxide ceramic base and a microporous layer on a silicon or other base.

11. System according to Claim 10, **characterized by** the fact that the microporous layer of the ceramic material of the membranes or the membrane wall is simultaneously made as a catalyst.

12. System according to one of Claims 6 through 11, **characterized by** the fact that the mass transfer device (4) can be operated using a heating or cooling medium as a flushing medium.

13. System according to one of Claims 6 through 12, **characterized by** the fact that the mass transfer device (4) is made with a gas-side bypass device (12).

## Revendications

1. Procédé pour le cracking thermique d'hydrocarbures, en particulier pour la préparation d'oléfines, du type selon lequel dans un four de cracking est chauffé un mélange d'hydrocarbures et de vapeur d'eau à une température de 750 °C à 900 °C, selon lequel immédiatement après le chauffage, le gaz de cracking contenant l'hydrogène alors formé est refroidi dans un réfrigérant de gaz de cracking à une température inférieure à 650 °c environ, puis encore refroidi par trempe lors de la présence de produits lourds et subséquemment libéré par séparation d'avec la fraction lourde de pétrole et l'essence, tandis qu'ensuite la vapeur d'eau est éliminée par trempe du gaz de cracking contenant des produits légers, qui est alors comprimé dans un compresseur à au moins 20 bars, libéré de l'eau restante par séchage, refroidi dans une installation de réfrigération poussée et conduit dans plusieurs colonnes de séparation afin d'en séparer les produits légers, et du type selon lequel dans le sens d'écoulement du fluide de process et en amont de l'installation de réfrigération poussée est extraite du gaz de cracking une partie de l'hydrogène au moyen d'une diffusion sélective à travers une membrane, ledit procédé étant **caractérisé en ce que** l'extraction de l'hydrogène hors du gaz de cracking s'effectue dans le sens d'écoulement du fluide de process et en amont du compresseur.

2. Procédé selon la revendication 1, **caractérisé en ce que** l'extraction de l'hydrogène hors du gaz de cracking s'effectue directement après son refroidissement dans le réfrigérant de gaz de cracking.

3. Procédé selon l'une des revendications précitées, **caractérisé en ce qu'**un fluide d'aspersion réduit la pression partielle de l'hydrogène extrait et élimine celle-ci.

4. Procédé selon l'une des revendications précédentes, **caractérisé en ce qu'**en tant que fluide d'aspersion est mis en oeuvre un fluide caloporteur ou un fluide réfrigérant, qui provoque simultanément à l'extraction de l'hydrogène hors du gaz de cracking, un réchauffage ou une réfrigération du gaz de cracking, par échange thermique.

5. Procédé selon l'une des revendications précédentes, **caractérisé en ce que** le gaz de cracking peut être introduit, par un dispositif de by-pass, dans le dispositif d'extraction de l'hydrogène.

6. Installation de cracking thermique d'hydrocarbures, en particulier pour la préparation d'oléfines, qui présente dans le sens d'écoulement du fluide de process:
un four de cracking (2) pour le chauffage d'un mélange d'hydrocarbures et de vapeur d'eau à une température de 750 °C à 900 °C en vue de la production de gaz de cracking,
un réfrigérant de gaz de cracking (3) pour le refroidissement du gaz de cracking à une température inférieure à 650 °C environ,
en cas de présence de produits lourds dans le gaz de cracking, un dispositif de trempe (5) pour condenser les produits lourds ainsi que des colonnes de séparation (6) pour séparer la fraction lourde de pétrole et l'essence,
une colonne de trempe (7) pour l'élimination de la vapeur d'eau ou de l'eau,
un compresseur (8) pour comprimer le gaz de cracking à au moins 20 bars,
un sécheur (9) pour éliminer l'eau restante,
une installation de réfrigérant poussée (10) et
plusieurs colonnes de séparation (11) pour séparer les produits légers,
et du type dans laquelle est disposé, dans le sens d'écoulement du fluide de process et en amont de l'installation de réfrigération poussée (10), un échangeur de matière (4) fonctionnant selon le principe de la diffusion au travers d'une membrane et destiné à l'extraction sélective d'hydrogène hors du gaz de cracking, ladite installation étant **caractérisée en ce que** l'échangeur de matière (4) est disposé dans le sens d'écoulement du fluide de process et en amont du compresseur (8).

7. Installation selon la revendication 6, **caractérisée en ce que** l'échangeur de matière (4) est directement raccordé avec le réfrigérant de gaz de cracking (3).

8. Installation selon la revendication 6 ou 7, **caractérisée en ce que** l'échangeur de matière (4) présente au moins un canal d'écoulement (15) et dans tous les cas au moins une entrée et une sortie (17, 18) pour le gaz de cracking, au moins un canal d'écoulement (16) et dans tous les cas au moins une entrée et une sortie (19, 20) pour un fluide d'aspersion, ainsi qu'une cloison formant membrane (14), séparant le gaz de cracking et le fluide d'aspersion, commune aux canaux d'écoulement (15, 16) et constituant au moins une partie de la paroi du canal d'écoulement (15, 16), tandis qu'au moins une section de la cloison formant membrane (14) est conformée afin de permettre de réaliser la diffusion d'hydrogène.

9. Installation selon la revendication 8, **caractérisée en ce que** la membrane, respectivement la cloison formant membrane, de l'échangeur de matière (4) est constituée de matériau céramique.

10. Installation selon la revendication 9, **caractérisée en ce que** la membrane, respectivement la cloison formant membrane, constituée de matériau céramique est constituée d'un matériau macroporeux à base d'alumine ou d'une autre céramique à base d'oxyde, ainsi que d'une couche microporeuse à base de silicium ou d'un autre composé.

11. Installation selon la revendication 10, **caractérisée en ce que** la couche microporeuse du matériau céramique de la membrane, respectivement de la cloison formant membrane, est en même temps conformée en tant que catalyseur.

12. Installation selon l'une des revendications 6 à 11, **caractérisée en ce que** l'échangeur de matière (4) est susceptible de fonctionner avec un fluide caloporteur ou un fluide réfrigérant en tant que fluide d'aspersion.

13. Installation selon l'une des revendications 6 à 12, **caractérisée en ce que** l'échangeur de matière (4) est conformé avec un dispositif de by-pass (12), situé côté gaz.
